## Europäisches Patentamt

## European Patent Office

## Office européen des brevets

(11) Veröffentlichungsnummer: **0 259 507**
**B1**

(12) # EUROPÄISCHE PATENTSCHRIFT

(45) Veröffentlichungstag der Patentschrift:
**23.08.89**

(51) Int. Cl.⁴: **A61N 5/06**

(21) Anmeldenummer: **86112390.9**

(22) Anmeldetag: **08.09.86**

(54) Vorrichtung zur balneo-phototherapeutischen Hautbehandlung.

(43) Veröffentlichungstag der Anmeldung:
**16.03.88 Patentblatt 88/11**

(45) Bekanntmachung des Hinweises auf die Patenterteilung:
**23.08.89 Patentblatt 89/34**

(84) Benannte Vertragsstaaten:
**AT BE CH DE FR IT LI LU NL**

(56) Entgegenhaltungen:
**EP-A- 0 221 199**
**DE-A- 2 910 468**
**DE-A- 3 317 140**

(73) Patentinhaber: **Schneider, Karl, Ostlandstrasse 12,
D-6427 Bad Salzschlirf(DE)**

(72) Erfinder: **Schneider, Karl, Ostlandstrasse 12, D-6427 Bad
Salzschlirf(DE)**

(74) Vertreter: **Linser, Heinz et al, Patentanwälte Heinz
Linser Dipl. Ing. Eckhardt Eyer
Robert-Bosch-Strasse 12a Postfach 10 22 10,
D-6072 Dreieich(DE)**

## Beschreibung

Die Erfindung betrifft eine Vorrichtung zur balneo-phototherapeutischen Behandlung von Haut- oder rheumatischen Erkrankungen und/oder kosmetischen oder prophylaktischen Hautbehandlungen mit einer Badeinrichtung und einer Bestrahlungsanlage, die aus einer Kombination einer Badewanne mit mindestens einem Strahler besteht und eine Badeflüssigkeit aufweist, die im Kreislauf die Badewanne, eine Filtereinrichtung, eine Entkeimungseinrichtung, eine Pumpe sowie eine Heizung und gegebenenfalls eine Aufbereitungsvorrichtung durchströmt und die Bestrahlungsanlage räumlich über der Badewanne und/oder unter der Liegefläche in der Badewanne angeordnet ist.

Zur Behandlung der Psoriasis und ähnlicher Hautkrankheiten ist es bekannt, einen Ausschnitt des UV-Spektrums im Grenzbereich von UVB und UVA zu verwenden, der Wellenlängen von 290 bis 330 nm aufweist.

Nach früherer Auffassung hat eine Strahlung, die länger als 330 nm ist, keine antipsoriatische Wirksamkeit mehr. Dies ist im Grundsatz zwar richtig, jedoch bringt die längerwellige Strahlung positive ergänzende Wirkungen, auf die später näher eingegangen wird.

Dementsprechend sind auch Geräte bekannt, welche eine UV-Strahlungsvorrichtung mit einer Metall-Hochdrucklampe aufweisen, deren Füllung so gewählt ist, dass sich mehrere stark ausgeprägte Spektrallinien im Bereich zwischen 300 und 330 nm finden. Diese Lampe ist eine punktförmige Strahlungsquelle, mit der sich grössere Flächen erst aus ausreichend grosser Entfernung gleichmässig bestrahlen lassen.

Bedingt durch eine intensive Entwicklung auf dem Gebiet der Heliotherapie sind heute eine Vielzahl von Reaktionen des Organismus auf die Applikation von optischer Strahlung bekannt, die zum Teil über die Beeinflussung des vegetativen Nervensystems und zum Teil durch photochemische Reaktionen in der Haut zustande kommen.

Entscheidend für alle diese Reaktionsabläufe sind die spektrale Verteilung der Strahlungsenergie und die Strahlendosis.

Während die langwellige Infrarot-Strahlung als ein wirksames Heilmittel bei Furunkulose, Rheumatismus und Erkältungen erkannt wurde, zeigt die sichtbare Strahlung über das Auge wirkend eine positive Beeinflussung der Psyche.

Besondere Bedeutung hat die therapeutische Anwendung von UV-Strahlung bei vielen Hautkrankheiten erreicht.

Hierbei ist der Wellenlängenbereich der optischen Strahlung von besonderer Bedeutung, der bei Absorption im Molekül Änderungen in der Elektronenschale bewirkt, ohne jedoch Ionisationsvorgänge einzuleiten. Positive Wirkungen können durch Strahlung im Wellenlängenbereich zwischen 300 bis 400 nm ausgelöst werden.

Kurzwelligere Strahlung als 290 nm sollte aus gesundheitlichen Gründen nicht eingesetzt werden und eine Strahlung, die langwelliger als 340 nm ist, zeigt allein eingesetzt keine Wirkung. In Kombination mit der Strahlung bis 300 nm liegen jedoch positive Wirkungen vor.

In den letzten Jahren wurden viele Bräunungsstudios eingerichtet, welche mit Sonnenbänken ausgerüstet sind, die zusätzlich mit einem auf- und abbewegbaren Sonnenhimmel ausgerüstet sein können, so daß eine Ganzkörperbestrahlung durchführbar ist. Eine übermäßige Strahlenbelastung kann Schäden hervorrufen, welche bis zur Hautkrebserkrankung führen können.

Seit Menschengedenken ist die für den Körper positive Wirkung eines Bades bekannt, insbesondere wenn das Bad mit Badesalzen angereichert und temperiert ist. Eine bevorzugte Stellung nimmt hier das Tote Meer ein.

Seine Heilwirkung ist bereits über tausende von Jahren bekannt, und bedingt durch seine große Anreicherung an Salzen der unterschiedlichsten Art, erstreckt sich die Heilwirkung auf die verschiedensten Krankheiten. Erst in jüngster Zeit wurden die in diesem Zusammenhang vorliegenden Probleme näher erforscht. Es stellt sich heraus, daß das Tote Meer eine andere Salzzusammensetzung aufweist als die übrigen Meere, wie etwa das Mittelmeer, die Nordsee oder bekannte Sole-Quellen. So ist insbesondere der Anteil an Magnesium, Kalium und Brom weitaus höher. Dies allein ist jedoch für die Erklärung der Heilwirkung noch nicht ausreichend.

Es wurde festgestellt, daß das Sonnenlicht im Bereich des Toten Meeres im Vergleich zur Globalstrahlung, d.h. die Strahlung, die direkt von der Sonne und gestreut von der Erdatmosphäre eine horizontale, ebene Erdoberfläche erreicht, erheblich abweicht. Es zeigt sich, daß die vergleichbaren Werte im Bereich von 300 bis 390 nm niedriger liegen als die Werte der Globalstrahlung. Bedingt durch die Lage des Toten Meeres ca. 400 m unter dem Meeresspiegel der übrigen Weltmeere ist für die Strahlung eine 400 m starke Luftschicht vorhanden, welche eine entsprechende Filterwirkung aufweist, wie sie an keiner anderen Stelle der Welt vorliegt. Im gleichen Verhältnis sind die Schwellenzeiten zum Erreichen der minimalen erythemwirksamen Dosis am Toten Meer grösser als bei der Globalstrahlung.

Der Erfindung liegt die Erkenntnis zugrunde, daß die Kombination der Wirkung des im Bereich des Toten Meeres herrschenden Lichtspektrums der Sonne mit der spezifischen Zusammensetzung des Meerwassers eine besondere Heilkraft für die verschiedensten Krankheiten, insbesondere Hautkrankheiten, entwickelt, wie sich durch wissenschaftliche Untersuchungen bestätigen lässt.

Der Erfindung liegt eine weitere Erkenntnis zugrunde, nämlich daß die negative Wirkung der in den Sonnenstudios verwendeten Sonnenbänke und Sonnenhimmel dadurch vermieden werden kann, daß die Haut während der Bestrahlung periodisch mit einer Flüssigkeit benetzt wird, welche in Wasser gelöste Salze und damit Ionen aufweist. Die durch die Ionen aktivierte Haut wird für die Strahlung sensibler und absorbiert diese.

Aus der EP-A 0 221 199, welche auf den Erfinder zurückgeht und gemäß Artikel 54 (3) EPÜ zu berücksichtigen ist, ist eine Vorrichtung zur balneo-phototherapeutischen Behandlung von Haut- oder rheumatischen Erkrankungen und/oder kosmetischen oder prophylaktischen Hautbehandlung mit einer Badeinrichtung und einer Bestrahlungsanlage bekannt, die aus einer Kombination einer Badewanne mit mindestens einem Strahler besteht und eine Badeflüssigkeit mit einer definierten Zusammensetzung aufweist, die im Kreislauf die Badewanne, eine Filtereinrichtung, eine Entkeimungseinrichtung, eine Pumpe sowie eine Heizung und gegebenenfalls eine Aufbereitungsvorrichtung durchströmt, wobei die Bestrahlungsanlage räumlich über der Badewanne und/oder unter einer Liegefläche in der Badewanne angeordnet ist. Die Vorrichtung ist ferner so ausgebildet, daß die Badewanne mit der Bestrahlungseinrichtung eine Einheit bildet, die horizontale Mittelachse der Bestrahlungseinrichtung und die horizontale Mittelachse der Badewanne zueinander parallel verlaufen und senkrecht übereinander liegen und der Abstand der horizontalen Achsen der Badewanne und der Bestrahlungseinrichtung durch Höhenverstellbarkeit der Bestrahlungsanlage veränderbar ist.

Der Erfindung liegt die Aufgabe zugrunde, eine solche Vorrichtung der oben genannten Art zu vervollkommnen, mit deren Hilfe eine einzelne Person einer Strahlung unterworfen wird, welche der Sonnenstrahlung nahe kommt, die am Toten Meer vorherrscht, in Kombination mit einem Bad, welches eine Zusammensetzung aufweist, die der Zusammensetzung des Wassers des Toten Meeres entspricht, wobei das Austrocknen der Haut durch Strahlenwirkung verhindert oder vermindert werden soll, und einer Vorrichtung zum sparsamen Einsatz der kostenaufwendigen Sole.

Die Lösung dieser Aufgabe besteht gemäß der Erfindung darin, daß bei der eingangs aufgeführten Vorrichtung die als Liegewanne ausgestaltete Badewanne derart ausgebildet ist, daß eine in der Badewanne befindliche und zu bestrahlende Person jede beliebige Lage einnehmen kann, und am Wannenauslauf eine ein Reinigungsfilter des Wasserkreislaufs enthaltende Einrichtung zur Füllstandsregelung vorgesehen ist, die die Füllstandshöhe in der Badewanne an die Wasserverdrängung der zu behandelnden Person angleicht, so daß eine der Strahlenquelle jeweils zugekehrte Seite der Person aus der Badeflüssigkeit ragt.

Mit dieser Vorrichtung nach der Erfindung ist es einer Einzelperson nunmehr möglich, sich gleichzeitig einem Bad, insbesondere einem Solebad spezifischer und einstellbarer Zusammensetzung in Kombination mit einer spezifischen Strahlung auszusetzen, wobei die Sole durch ständige oder periodische Umwälzung auf der erforderlichen Temperatur gehalten werden kann. Gleichzeitig wird kontinuierlich eine Entkeimung durchgeführt, so dass die Sole vielfach benutzt werden kann.

In Weiterbildung der Erfindung ist die Badewanne in ihrer Längsrichtung eben ausgebildet und weist eine Kopfstütze auf, welche gegebenenfalls höhenverstellbar ist.

Die Lampe-Reflektor-Filter-Einrichtung der Bestrahlungsanlage weist zur Anpassung an die jeweils gewünschte biologische Wirkungsfunktion in vorteilhafter Weise austauschbare Filter auf.

Die Verteilung der Strahler in Badewannenlängsrichtung ist nach der Erfindung derartig vorgesehen, daß sich eine gleichmäßige Verteilung der Bestrahlungsstärke in der Nutzebene der Badewanne ergibt.

In Weiterbildung der Erfindung weist die Bad- bzw. Solebadentkeimungseinrichtung eine UV-Einrichtung auf.

In Weiterbildung der Erfindung ist die Einrichtung zur Füllstandsregelung derartig ausgebildet, daß sie das Wasser oder die Sole von der Flüssigkeitsoberfläche dem Solekreislauf zuführt.

Ferner weist die Einrichtung zur Füllstandsregelung einen Thermoregler zur Steuerung der Heizung des Solekreislaufes auf.

Die Heizung im Solekreislauf ist in vorteilhafter Weise als elektrische Widerstandsheizung ausgebildet, welche im Durchflußverfahren die Sole auf die vom Thermoregler vorgegebene Temperatur erwärmt, wobei die Sole als Heizleiter der elektrischen Widerstandsheizung dient.

Die mit der Solebadvorrichtung als Einheit ausgebildete Bestrahlungseinrichtung beaufschlagt die größte horizontale Innenfläche der Therapiewanne mit einer Bestrahlungsstärke, daß die jeweils erforderliche Schwellenbestrahlung nach längstens 30 Minuten erreicht wird, wobei die Bestrahlungsstärkeverteilung in der Nutzebene möglichst gleichmäßig ist.

Die Erfindung wird anhand der Zeichnungen näher erläutert. Hierbei zeigen:

Figur 1 einen Querschnitt durch eine Badvorrichtung nach der Erfindung.

Figur 2 eine Draufsicht auf die Badvorrichtung nach Figur 1, und

Figur 3 eine vergrößerte Darstellung höhenveränderbaren Füllstandsüberlaufreglers.

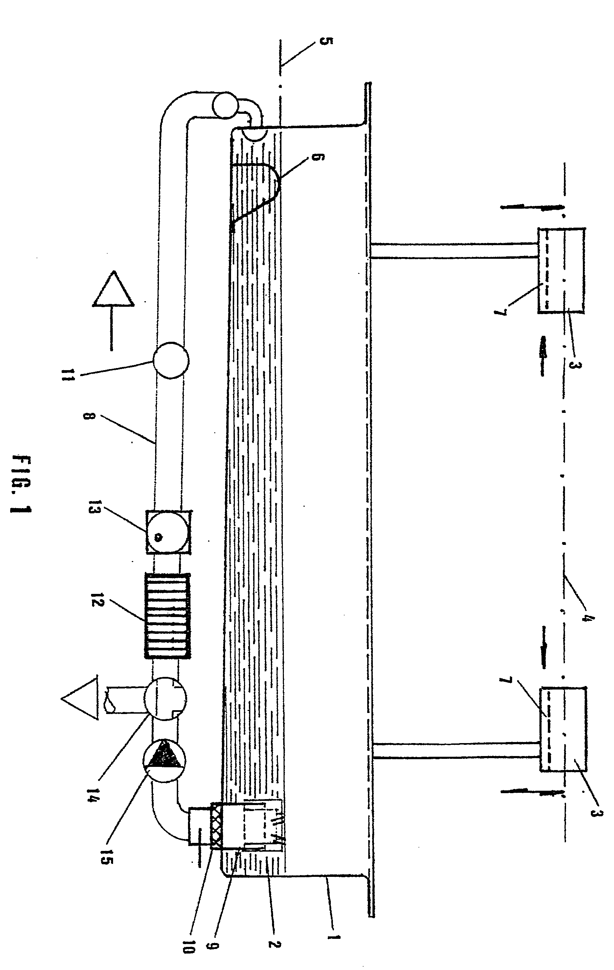

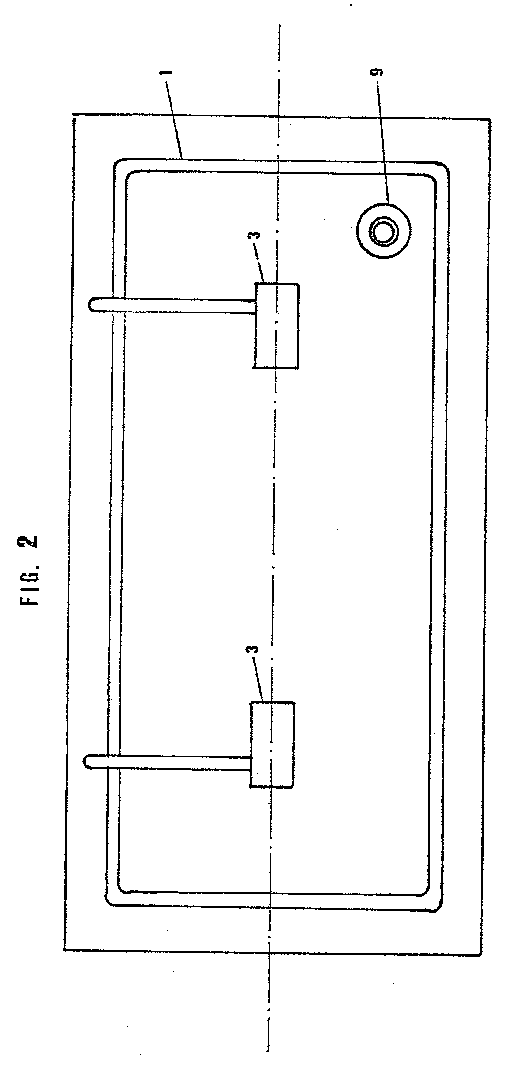

Wie aus den Figuren 1 und 2 ersichtlich ist, ist die als Liegewanne 1 ausgebildete Therapiewanne derart gestaltet, daß die in der Badewanne befindliche und zu bestrahlende Person jede beliebige Lage einnehmen kann, so daß die der Bestrahlungsvorrichtung 3 jeweils zugekehrte Seite der Person aus der Badeflüssigkeit 2, welche vorzugsweise aus einer Sole besteht, herausragt.

Die Badewanne 1 bildet mit der Bestrahlungseinrichtung 3 bzw. den Strahlenquellen eine Einheit, so daß sich diese hinsichtlich ihrer Strahlungsverteilung maximal anordnen und justieren lassen.

Hierzu verlaufen die horizontale Mittelachse 4 der gesamten Bestrahlungsvorrichtung 3 und die horizontale Mittelachse 5 der Badewanne 1 zueinander parallel und liegen senkrecht übereinander. Der Abstand der horizontalen Mittelachse 5 von der Badewanne 1 und der Mittelachse 4 der Bestrahlungsvorrichtung 3 ist durch Höhenverstellbarkeit der Bestrahlungsvorrichtung 3 veränderbar.

Die Badewanne 1 ist in ihrer Längsrichtung eben ausgebildet und weist eine Kopfstütze 6 auf. Diese kann, falls es erforderlich ist, höhenverstellbar sein.

Die spektrale Strahlungsflussverteilung der Lampe-Reflektor-Filter-Einrichtung der Bestrahlungsvorrichtung 3 weist zur Anpassung an die jeweils gewünschte biologische Wirkungsfunktion austauschbare Filter 7 auf.

Die Verteilung der Strahler der Bestrahlungsvorrichtung 3 oberhalb der Badvorrichtung 1 ist in Längsrichtung der Badewanne derartig ausgebildet, daß sich eine gleichmäßige Verteilung der Bestrahlungsstärke in der Nutzebene, das heißt kurz oberhalb des Flüssigkeitsspiegels der Badewanne 1 ergibt.

Zur ständigen Umwälzung der Badeflüssigkeit oder Sole 2 ist ein Kreislauf 8 vorgesehen, in dem ein Füllstandsregler 9, ein Filter 10, eine Pumpe 11, eine Heizung 12, eine Entkeimungsvorrichtung 13, ein vorzugsweise fernsteuerbarer Dreiwegehahn 14 und ein vorzugsweise fernsteuerbares Ventil 15 vorgesehen ist. Die Entkeimungseinrichtung 13 weist vorteilhaft eine UV-Einrichtung auf.

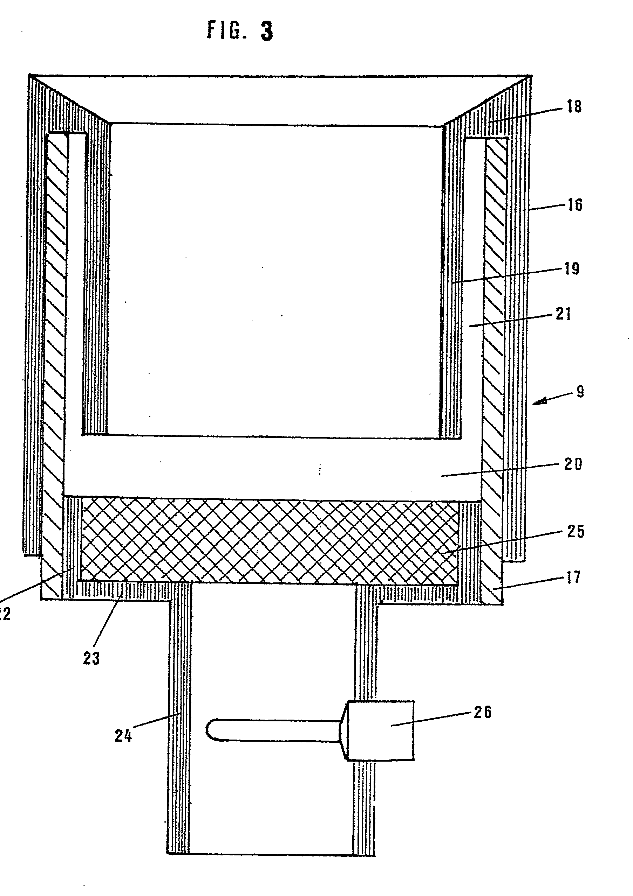

Die Figur 3 zeigt in vergrößerter Darstellung die Einrichtung zur Füllstandsregelung 9, welche die Füllstandshöhe in der Badewanne 1 an die Wasserverdrängung der zu behandelnden Person angleicht. Die Füllstandsregelung 9 ist am Wannenauslauf positioniert und enthält das Reinigungsfilter 10 des Wasserkreislaufes (welches in der Figur 3 mit 25 bezeichnet ist).

Die Einrichtung zur Füllstandsregelung 9 ist derartig ausgebildet, daß sie die Badeflüssigkeit von der Oberfläche dem Wasserkreislauf zuführt.

Die Füllstandsregelung 9 besteht aus einem äusseren Rohr 16, in dem ein weiteres Rohr 17 gleitbar gelagert ist. Mit dem Rohr 16 ist am oberen Ende ein Abschlußring 18 befestigt, mit dem rohrinnenseitig ein Rohr 19 verbunden ist, welches in seiner unteren Position einen Spalt 20 zum Luftmantel 21 offen läßt. Dieser Luftmantel 21 wird somit von dem Rohr 17, dem Abschlußring 18 und dem Rohr 19 begrenzt.

Ein Rohrstutzen 22 ist mit einem Bodenring 23 und dieser mit einem Auslaufrohr 24 befestigt, welches ein Gewinde aufweisen kann. In dem Rohrstutzen 22 ist ein Filter 25 (10) angeordnet, welches auf einer nicht näher dargestellten Lochscheibe gelagert ist. In dem Auslaufrohr 24 befindet sich ein Thermofühler 26.

Ist der Flüssigkeitsdurchfluß durch das Auslaufrohr 24 blockiert, so steigt die Flüssigkeit in dem Rohr 19 und in dem Raum des Luftmantels 21 auf. Diese Luft wird zusammengepreßt, erhöht ihren Druck und hebt den Abschlussring 18 mit dem damit verbundenen Rohr 19 an, so daß sich ein erhöhter Wasserspiegel in der Badewanne einstellt.

Die Heizung 12 liegt im Solekreislauf 8 und ist als elektrische Widerstandsheizung ausgebildet. Die Sole wird im Durchflußverfahren auf die vom Thermoregler vorgegebene Temperatur erwärmt, wobei die Sole als Heizleiter der elektrischen Widerstandsheizung dient.

**Patentansprüche**

1. Vorrichtung zur balneo-phototherapeutischen Behandlung von Haut- oder rheumatischen Erkrankungen und/oder kosmetischen oder prophylaktischen Hautbehandlung mit einer Badeinrichtung und einer Bestrahlungsanlage, die aus einer Kombination einer Badewanne (1) mit mindestens einem Strahler (3) besteht und eine Badeflüssigkeit aufweist, die im Kreislauf die Badewanne (1), eine Filtereinrichtung, eine Entkeimungseinrichtung (13), eine Pumpe (11) sowie eine Heizung (12) und gegebenenfalls eine Aufbereitungsvorrichtung durchströmt, wobei die Bestrahlungsanlage räumlich über der Badewanne (1) und/oder unter einer Liegefläche in der Badewanne (1) angeordnet ist, und die als Liegewanne ausgestaltete Badewanne (1) derart ausgebildet ist, daß eine in der Badewanne befindliche und zu bestrahlende Person jede beliebige Lage einnehmen kann, und am Wannenauslauf eine ein Reinigungsfilter (10, 25) des Wasserkreislaufs enthaltende Einrichtung zur Füllstandsregelung (9) vorgesehen ist, die die Füllstandshöhe in der Badewanne an die Wasserverdrängung der zu behandelnden Person angleicht, so dass eine der Strahlenquelle (3) jeweils zugekehrte Seite der Person aus der Badeflüssigkeit (2) ragt, und die Vorrichtung ferner so ausgebildet ist, daß die Badewanne (1) mit der Bestrahlungseinrichtung (3) eine Einheit bildet, die horizontale Mittelachse (4) der Bestrahlungseinrichtung (3) und die horizontale Mittelachse (5) der Badewanne zueinander parallel verlaufen und senkrecht übereinander liegen und der Abstand der horizontalen Achsen der Badewanne (1) und der Bestrahlungseinrichtung (3) durch Höhenverstellbarkeit der Bestrahlungsanlage veränderbar ist.

2. Vorrichtung nach Anspruch 1, dadurch gekennzeichnet, daß die Badewanne (1) in ihrer Längsrichtung eben ausgebildet ist und eine Kopfstütze (6) aufweist, welche gegebenenfalls höhenverstellbar ist.

3. Vorrichtung nach Anspruch 1 oder 2, dadurch gekennzeichnet, daß die Lampe-Reflektor-Filter-Einrichtung der Bestrahlungsanlage (3) zur Anpassung an die jeweils gewünschte biologische Wirkungsfunktion austauschbare Filter (7) aufweist.

4. Vorrichtung nach Anspruch 1, 2 oder 3, dadurch gekennzeichnet, daß die Verteilung der Strahler (3) in Badewannenlängsrichtung derartig vorgesehen ist, daß sich eine gleichmäßige Verteilung der Bestrahlungsstärke in der Nutzebene der Badewanne ergibt.

5. Vorrichtung nach Anspruch 1 oder einem der voranstehenden Ansprüche, dadurch gekennzeichnet, daß die Badentkeimungseinrichtung eine UV-Einrichtung aufweist.

6. Vorrichtung nach Anspruch 1 oder einem der voranstehenden Ansprüche, dadurch gekennzeichnet, daß die Einrichtung zur Füllstandsregelung (9) derartig ausgebildet ist, daß sie die Badeflüssigkeit von der Oberfläche dem Wasserkreislauf zuführt.

7. Vorrichtung nach Anspruch 1 oder einem der voranstehenden Ansprüche, dadurch gekennzeichnet, daß die Einrichtung zur Füllstandsregelung (9) einen Thermoregler (26) zur Steuerung der Heizung des Wasserkreislaufes enthält.

8. Vorrichtung nach Anspruch 1 oder einem der voranstehenden Ansprüche, dadurch gekennzeichnet, daß das Badewasser eine Sole aufweist und die Heizung (12) im Solekreislauf als elektrische Widerstandsheizung ausgebildet ist, welche im Durchflußverfahren die Sole auf die vom Thermoregler vorgegebene Temperatur erwärmt, wobei die Sole als Heizleiter der elektrischen Widerstandsheizung dient.

9. Vorrichtung nach Anspruch 1, oder einem der voranstehenden Ansprüche, dadurch gekennzeichnet, daß die mit der Badvorrichtung als Einheit ausgebildete Bestrahlungseinrichtung (3) die größte horizontale Innenfläche der Badewanne (1) mit einer solchen Bestrahlungsstärke beaufschlagt, daß die jeweils erforderliche Schwellenbestrahlung nach längstens 30 Minuten erreicht wird, wobei die Bestrahlungsstärkeverteilung in der Nutzebene möglichst gleichmäßig ist.

**Claims**

1. A device for balneo-phototherapeutic treatment of skin or rheumatic disorders and/or cosmetic or prophylactic skin treatment with a bath arrangement and an irradiating arrangement which consists of a combination of a bath (1) with at least one radiator (3) and exhibits a bath liquid which is circulated through the bath (1), a filtering arrangement, a disinfecting arrangement (13), a pump (11) an a heater (12) and possibly a processing device, the irradiating arrangement being disposed spatially above the bath (1) and/or under a reclining surface in the bath (1), and the bath (1) in the form of a reclining bath is so constituted that a person in the bath and to be irradiated can adopt any desired position, and a level regulating arrangement (9) comprising a cleaning filter (10, 25) for the water circuit is provided at the bath outlet, which arrangement adjusts the level in the bath according to the water displaced by the person to be treated so that the side of the person facing the radiation source (3) projects from the bath liquid (2), and the device is also so formed that the bath (1) forms a unit with the irradiating arrangement (3), the horizontal median axis (4) of the irradiating arrangement (3) and the horizontal median axis (5) of the bath run parallel to one another and lie perpendicularly above one another and the distance between the horizontal axes of the bath (1) and the irradiating arrangement (3) can be altered by adjusting the height of the irradiating arrangement.

2. A device as in claim 1, characterised in that the bath (1) is flat in its longitudinal direction and exhibits a head rest (6) which is adjustable in height if necessary.

3. A device as in claim 1 or 2, characterised in that the lamp-reflector-filter-arrangement of the irradiating arrangement (3) exhibits interchangeable filters (7) for adjustment to the particular desired effective biological action.

4. A device as in claim 1, 2 or 3, characterised in that the distribution of the radiators (3) in the longitudinal direction of the bath is such as to produce uniform distribution of the radiation in the working plane of the bath.

5. A device as in claim 1 or one of the preceding claims, characterised in that the bath disinfecting arrangement exhibits a UV arrangement.

6. A device as in claim 1 or one of the preceding claims, characterised in that the level regulating arrangement (9) is so formed that it feeds the bath liquid from the surface to the water circuit.

7. A device as in claim 1 or one of the preceding claims, characterised in that the level regulating arrangement (9) comprises a temperature controller (26) to control the heating of the water circuit.

8. A device as in claim 1 or one of the preceding claims, characterised in that the bath water exhibits a brine and the heating (12) in the brine circuit takes the form of electric resistance heating which heats the brine to the temperature preset by the temperature controller as it flows through, the brine serving as heating conductor for the electric resistance heating.

9. A device as in claim 1 or one of the preceding claims, characterised in that the irradiating arrangement (3) forming a unit with the bath arrangement irradiates the largest horizontal internal surface of the bath (1) with such an intensity that the required threshold radiation level is reached after at most 30 minutes and the radiation intensity distribution in the working plane is as uniform as possible.

**Revendications**

1. Dispositif pour le traitement balnéo-phototherapeutique d'affections cutanées ou rhumatismales et/ou le traitement cosmétique ou prophylactique de la peau au moyen d'une installation de bains et d'un installation d'irradiation qui combine une baignoire (1) avec au moins une source de rayonnement (3) et présente un liquide de bain qui parcourt, selon un mouvement circulaire, la baignoire (1), une installation de filtration, une installation de désinfection (13), une pompe (11), ainsi qu'un chauffage (12) et, éventuellement, un dispositif de préparation, dans lequel l'installation d'irradiation est disposée spatialement au-dessus de la baignoire (1) et/ou en-dessous d'une surface de repos dans la baignoire (1), et la baignoire (1) ayant la forme d'une baignoire allongée est configurée de manière telle qu'une personne se trouvant dans la baignoire et destinée à être exposée rayonnement peut prendre une position quelconque, et un dispositif de régulation du niveau (9) comportant un filtre de nettoyage (10, 25) du circuit de l'eau est prévu à la sortie de la baignoire, lequel dispositif adapte la hauteur du niveau dans la baignoire au déplacement de la personne à traiter, de façon telle qu'un côté de la personne tourné vers la source de rayonnement (3) sort du liquide du bain (2), et le dispositif est, en outre, configuré de manière telle que la baignoire (1) forme avec l'unité d'irradiation (3) un ensemble, l'axe horizontal (4) de l'installation d'irradiation (3) et l'axe horizontal (5) de la baignoire sont parallèles l'un à l'autre et se trouvent perpendiculairement l'un au-dessus de l'autre, et la distance des axes horizontaux de la baignoire (1) et de l'installation d'irradiation (3) est modifiable par la possibilité de réglage en hauteur de l'installation d'irradiation.

2. Dispositif selon la revendication 1, caractérisé en ce que la baignoire (1) est plane dans le sens longitudinal, et présente un appuitête (6), lequel peut être éventuellement réglé en hauteur.

3. Dispositif selon la revendication 1 ou 2, caractérisé en ce que l'installation lampe-réflecteur-filtre de l'installation d'irradiation (3) présente des filtres interchangeables (7) pour une adaptation chacune des fonctions d'activation biologique désirées.

4. Dispositif selon la revendication 1, 2 ou 3, caractérisé en ce que la répartition des sources de rayonnement (3) dans le sens longitudinal de la baignoire est prévu de manière telle que l'on obtient une répartition uniforme de la puissance de rayonnement dans le plan utile de la baignoire.

5. Dispositif selon la revendication 1 ou une des revendications qui précèdent, caractérisé en ce que l'installation de désinfection du bain présente une installation UV.

6. Dispositif selon la revendication 1, ou une des revendications qui précèdent, caractérisé en ce que l'installation de régulation du niveau (9) est configurée de sorte telle qu'elle amène le liquide du bain depuis la surface au circuit de l'eau.

7. Dispositif selon la revendication 1, ou une des revendications qui précèdent, caractérisé en ce que l'installation de régulation du niveau (9) comporte un thermorégulateur (26) pour la commande du chauffage du circuit de l'eau.

8. Dispositif selon la revendication 1 ou une des revendications qui précèdent, caractérisé en ce que l'eau du bain présente une saumure et le chauffage (12) dans le circuit de la saumure a la forme d'un chauffage à résistance électrique qui réchauffe par le procédé à circulation la saumure à la température pré-réglée par le thermorégulateur, la saumure servant de conducteur chauffant pour le chauffage à résistance électrique.

9. Dispositif selon la revendication 1 ou une des revendications qui précèdent, caractérisé en ce que le dispositif d'irradiation (3) formant une unité avec l'installation de bain alimente la plus grande surface intérieure horizontale de la baignoire (1) avec une intensité de rayonnement telle que le rayonnement de seuil nécessaire dans chaque cas est atteint au plus tard après 30 minutes, la réparation de la force de rayonnement étant la plus uniforme possible dans le plan utile.

FIG. 1

# FIG. 2

EP 0 259 507 B1

## FIG. 3